# EUROPEAN PATENT SPECIFICATION

(11) **EP 4 477 175 B1**
(45) Date of publication and mention of the grant of the patent: **25.11.2026**
(21) Application number: 24177077.5
(22) Date of filing: 21.05.2024
(51) Int. Cl.: A61B 34/20, A61B 34/10

(54) **MEDICAL IMAGING METHOD, APPARATUS, AND SYSTEM**
VERFAHREN, VORRICHTUNG UND SYSTEM ZUR MEDIZINISCHEN BILDGEBUNG
PROCÉDÉ, APPAREIL ET SYSTÈME D'IMAGERIE MÉDICALE

(30) Priority: 31.05.2023 CN 202310636115
(43) Date of publication of application: 18.12.2024
(73) Proprietor: GE Precision Healthcare LLC, Waukesha, WI 53188 (US)
(72) Inventor: LI, Feng, Milwaukee, 53226 (US)
(74) Representative: Kilburn & Strode LLP

(56) References cited:
- US-A1- 2002 198 518
- US-A1- 2018 185 113
- US-A1- 2019 110 685
- US-A1- 2021 085 268
- US-A1- 2023 157 765
- US-B2- 9 610 134

## Description

### TECHNICAL FIELD

Embodiments of the present application relate to the technical field of medical devices, and in particular to a medical imaging method, a storage medium and a computer device.

### BACKGROUND

During interventional procedures, a medical accessory must be inserted into a subject at a correct position and angle, so as to ensure that the medical accessory can accurately arrive at a target position inside the subject. Since an operator cannot directly observe the internal structure of the subject, the operator cannot confirm in real time the position of the medical accessory inside the subject.

To confirm the position of the medical accessory inside the subject during an interventional procedure, it is often necessary to scan the subject many times, and for the operator to adjust the position of the medical accessory inside the subject many times according to the result of each scan, so that the medical accessory ultimately arrives at the target position. Each adjustment of the position of the medical accessory inside the subject mainly depends on the experience of the operator, so the accuracy and reliability of the interventional procedure cannot be ensured. In addition, since multiple scans are required during the interventional procedure, the entire interventional procedure is complicated and time-consuming.

It should be noted that the above introduction of the background is only set forth to help clearly and completely describe the technical solutions of the present application, and to facilitate the understanding of those skilled in the art.

US 2018/185113 A1 describes, according to its abstract, methods and systems for performing computer-assisted image-guided surgery, including robotically-assisted surgery. A method of displaying image data includes displaying image data of a patient on a handheld display device, tracking the handheld display device using a motion tracking system, and modifying the image data displayed in response to changes in the position and orientation of the handheld display device. Further embodiments include a sterile case for a handheld display device, display devices on a robotic arm, and methods and systems for performing image-guided surgery using multiple reference marker devices fixed to a patient.

US 2019/110685 A1 describes, according to its abstract, a system that includes an interventional device, a tracking device, an optical tracking system, at least one processor, and a display unit. The interventional device includes an insertion portion and an exterior portion. The insertion portion is configured for use inside of a patient to perform an interventional procedure. The tracking device is disposed proximate to the exterior portion of the interventional device. The optical tracking system is configured to cooperate with the tracking device to provide tracking imaging corresponding to a location and orientation of the tracking device. At least one processor is configured to correlate the tracking imaging information with an anatomical image of the patient to provide a combined image during a medical task for which at least a portion of the insertion portion of the interventional device is disposed inside of the patient. The display unit is configured to display the combined image.

US 2002/198518 A1 describes, according to its abstract, an entry position grid for aiding a user in locating the approximate entry points of a plurality of cryoprobes into a patient, wherein the cryoprobes are inserted into the perineal area of the patient and then into the prostate, the entry position grid including a frame portion having a center longitudinal axis, a grid portion extending across the frame portion, the grid portion defining a plurality of evenly spaced discrete openings, identifying indicia located on the frame that identifies each discrete opening, each discrete opening being adapted to allow the user to position the cryoprobes through the entry position grid and into the patient at an angle that is not parallel to the center axis, the grid portion comprising a continuous strand of flexible material that is attached to the frame portion.

US 2021/085268 A1 describes, according to its abstract, systems and methods for image-guided medical procedures use fluoroscopic 3D reconstructions to plan and navigate a percutaneously-inserted device such as a biopsy tool from an entry point to a target.

### SUMMARY

Provided in embodiments of the present application is a medical imaging method with further examples describing a corresponding apparatus and system.

According to one aspect of the embodiments of the present application, a medical imaging method is provided, which comprises:
Determining the position of a medical accessory in a reference coordinate system based on a camera image acquired by an auxiliary camera;
Reconstructing a medical image according to a scanning result and based on the reference coordinate system; and
Displaying in real time the position of the medical accessory in the medical image;
Generating a grid matrix according to the scanning result, wherein a starting point and/or a target point of a planned movement path of the medical accessory are/is marked in the grid matrix; and
Superimposing the grid matrix in the camera image.

According to another aspect of the present disclosure, a medical imaging apparatus is provided, comprising:
A determination unit which determines the position of a medical accessory in a reference coordinate system based on a camera image acquired by an auxiliary camera;
A reconstruction unit which reconstructs a medical image according to a scanning result and based on the reference coordinate system; and
A display unit which displays in real time the position of the medical accessory in the medical image.

According to one aspect of the present disclosure, a medical imaging system is provided, comprising:
A processor which determines the position of a medical accessory in a reference coordinate system based on a camera image acquired by an auxiliary camera, and reconstructs a medical image according to a scanning result and based on the reference coordinate system; and
A display which displays in real time the position of the medical accessory in the medical image.

One of the beneficial effects of the embodiments of the present application is that: the position of the medical accessory in the reference coordinate system is determined based on the camera image acquired by the auxiliary camera, and the medical image is reconstructed according to the scanning result and based on the reference coordinate system. Therefore, the position of the medical accessory in the medical image can be displayed in real time, so that the convenience of a procedure can be improved, the positioning process of the medical accessory can be simplified and shortened, and the operator can quickly and accurately move the medical accessory to the target position.

With reference to the following description and drawings, specific implementations of the embodiments of the present application are disclosed in detail, and the means by which the principles of the embodiments of the present application can be employed are illustrated. It should be understood that the implementations of the present application are not limited in scope thereby. Within the scope of the appended claims, the implementations of the present application comprise many changes, modifications, and equivalents.

### BRIEF DESCRIPTION OF THE DRAWINGS

The included drawings are used to provide further understanding of the embodiments of the present application, which constitute a part of the description and are used to illustrate the implementations of the present application and explain the principles of the present application together with textual description. Evidently, the drawings in the following description are merely some embodiments of the present application, and a person of ordinary skill in the art may obtain other implementations according to the drawings without involving inventive skill. In the drawings:
FIG. 1 is a schematic diagram of a CT imaging device according to the disclosure;
FIG. 2 is a schematic diagram of a medical imaging method according to an embodiment of the present application;
FIG. 3 is a schematic diagram of a medical imaging system according to the disclosure;
FIG. 4 is a schematic diagram of an example of 201 according to an embodiment of the present application;
FIG. 5 is a schematic diagram of an example of 401 according to an embodiment of the present application;
FIG. 6 is a schematic diagram of a method for determining a first transformation matrix according to an embodiment of the present application;
FIG. 7 is a schematic diagram of a calibration tool according to the disclosure;
FIG. 8 is a schematic diagram of a scanning coordinate system and a world coordinate system according to an embodiment of the present application;
FIG. 9 is a schematic diagram of a displayed image according to an embodiment of the present application;
FIG. 10 is another schematic diagram of a displayed image according to an embodiment of the present application;
FIG. 11 is another schematic diagram of a displayed image according to an embodiment of the present application;
FIG. 12 is another schematic diagram of a medical image imaging method according to an embodiment of the present application;
FIG. 13 is a schematic diagram of a medical imaging apparatus according to the disclosure;
FIG. 14 is a schematic diagram of a computer device according to the disclosure;
FIG. 15 is a schematic diagram of a medical imaging system according to the disclosure; and
FIG. 16 is a schematic diagram of a CT system according to the disclosure.

### DETAILED DESCRIPTION

The foregoing and other features of the embodiments of the present application will become apparent from the following description and with reference to the drawings. In the description and drawings, specific implementations of the present application are disclosed in detail, and part of the implementations in which the principles of the embodiments of the present application may be employed are indicated. It should be understood that the present application is not limited to the described implementations. On the contrary, the embodiments of the present application include all modifications, variations, and equivalents which fall within the scope of the appended claims.

In the embodiments of the present application, the terms "first" and "second" and so on are used to distinguish different elements from one another by their title, but do not represent the spatial arrangement, temporal order, or the like of the elements, and the elements should not be limited by said terms. The term "and/or" includes any one of and all combinations of one or more associated listed terms. The terms "comprise", "include", "have", etc., refer to the presence of stated features, elements, components, or assemblies, but do not exclude the presence or addition of one or more other features, elements, components, or assemblies.

In the embodiments of the present application, the singular forms "a" and "the" or the like include plural forms, and should be broadly construed as "a type of" or "a kind of" rather than being limited to the meaning of "one". Furthermore, the term "the" should be construed as including both the singular and plural forms, unless otherwise specified in the context. In addition, the term "according to" should be construed as "at least in part according to...", and the term "based on" should be construed as "at least in part based on...", unless otherwise clearly specified in the context.

The features described and/or illustrated for one embodiment may be used in one or more other embodiments in an identical or similar manner, combined with features in other embodiments, or replace features in other embodiments. The term "include/comprise" when used herein refers to the presence of features, integrated components, steps, or assemblies, but does not exclude the presence or addition of one or more other features, integrated components, steps, or assemblies.

The device for obtaining medical image data (such as a scanning result) described herein may be suitable for various medical imaging modalities, including but not limited to a computed tomography (CT) device, a magnetic resonance imaging (MRI) device, an ultrasound imaging device, a positron emission computed tomography (PET) device, a single photon emission computed tomography (SPECT) device, a PET/CT, a PET/MR, or any other suitable medical imaging devices.

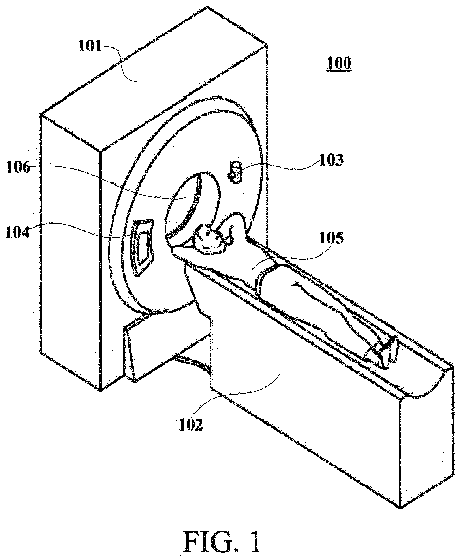

For example, CT uses X-rays to perform continuous profile scanning around a certain part of a scanned subject, and the X-rays that pass through a section are received by a detector and transformed into visible light, or a received photon signal is directly converted to form medical image data after a series of processing. FIG. 1 is a schematic diagram of a CT imaging device according to the disclosure, schematically showing a CT imaging device 100. Referring to FIG. 1, the CT imaging device 100 includes a scanning gantry 101 and a patient table 102. The scanning gantry 101 has an X-ray source 103, and the X-ray source 103 projects an X-ray beam toward a detector assembly or collimator 104 on an opposite side of the scanning gantry 101. A test subject 105 can lie flat on the patient table 102 and be moved into a scanning gantry opening 106 along with the patient table 102. Medical image data of the test subject 105 can be acquired by scanning performed by the X-ray source 103. The present application is not limited to this, and the CT imaging device 100 may be another structure.

MRI forms an image by means of reconstruction, based on the principle of nuclear magnetic resonance of atomic nuclei, by transmitting radio frequency pulses to the scanned subject and receiving electromagnetic signals released by the scanned subject.

PET uses a cyclotron to accelerate charged particles to bombard a target nucleus, which produces positron-bearing radionuclides by means of nuclear reactions and synthesizes imaging agents that are introduced into the body and localized in a target organ. The radionuclides emit positively charged electrons during a decay process, and after a positron travels a short distance in the tissue, the positron interacts with the electrons in the surrounding material and annihilation radiation occurs, from which two photons of equal energy are emitted in opposite directions. PET imaging uses a series of paired detectors that are arranged 180 degrees from each other and that receive coincidence lines to detect the photons of annihilating radiation produced by a tracer outside the body, and the collected information is processed by a computer to obtain a reconstructed image.

SPECT uses a radioactive isotope as a tracer, and the tracer is injected into the human body so that the tracer is concentrated on an organ to be examined, thus making the organ a source of γ-rays, and the distribution of radioactivity in organ tissue is recorded outside the body using detectors that rotate around the human body. One set of data is obtained when the detectors rotate to one angle, and several sets of data can be obtained when the detectors rotate a full circle. From said data, a series of tomographic planar images can be created, and a computer reconstructs the imaging in a cross-sectional manner.

PET and SPECT extend histopathological examination from a molecular level to display of local biochemistry of a tissue, and the provided images are images of human physiological metabolism, which are good at functional imaging and can detect functional and metabolic changes in the disease occurrence and development process; while CT and MRI are good at accurately reflecting morphological and structural changes. In existing methods, CT or MRI may be used for attenuation correction of PET or SPECT images. That is, PET or SPECT and CT or MRI are fused into one, so that functional and anatomical image information can complement one other to achieve better recognition and diagnosis.

The system obtaining the medical image data may include the aforementioned medical imaging device, and/or a separate computer device connected to the medical imaging device, and/or a computer device connected to an Internet cloud. The computer device may be connected by means of the Internet to the medical imaging device or a memory for storing medical images. An imaging method may be independently or jointly implemented by the aforementioned medical imaging device, the computer device connected to the medical imaging device, and the computer device connected to the Internet cloud.

In addition, a medical imaging workstation may be disposed locally at the medical imaging device. That is, the medical imaging workstation is disposed near to the medical imaging device, and the medical imaging workstation and medical imaging device may be located together in a scanning room, an imaging department, or the same hospital. In contrast, a medical image cloud platform analysis system may be positioned distant from the medical imaging device, e.g., arranged at a cloud end that is in communication with the medical imaging device.

As an example, after a medical institution completes an imaging scan using the medical imaging device, data obtained by scanning is stored in a storage device. A medical imaging workstation may directly read the data obtained by scanning and perform image processing by means of a processor thereof. As another example, the medical image cloud platform analysis system may read a medical image in the storage device by means of remote communication to provide "software as a service (SaaS)." SaaS can exist between hospitals, between a hospital and an imaging center, or between a hospital and a third-party online diagnosis and treatment service provider.

In the embodiments of the present application, the term "subject" may be equivalently replaced with "scanning subject", "subject to be scanned", "scanned subject", "test subject", "subject to be tested" and "tested subject", which may include any imaged subject. The terms "pixel" and "voxel" may be used interchangeably. The terms "layer" and "section" may be used interchangeably.

The following is a specific description of embodiments of the present application with reference to the drawings.

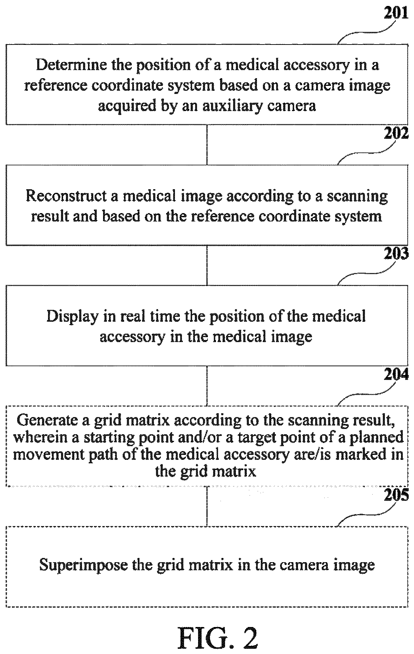

Provided in the embodiments of the present application is a medical imaging method. FIG. 2 is a schematic diagram of a medical imaging method according to an embodiment of the present application. As shown in FIG. 2, the medical imaging method includes:
201: Determining the position of a medical accessory in a reference coordinate system based on a camera image acquired by an auxiliary camera;
202: Reconstructing a medical image according to a scanning result and based on the reference coordinate system; and
203: Displaying in real time the position of the medical accessory in the medical image.

According to said embodiment, the position of the medical accessory in the reference coordinate system is determined based on the camera image acquired by the auxiliary camera, and the medical image is reconstructed according to the scanning result and based on the reference coordinate system. Therefore, the position of the medical accessory in the medical image can be displayed in real time, so that the convenience of the procedure can be improved, the positioning process of the medical accessory can be simplified and shortened, and the operator can quickly and accurately move the medical accessory to the target position.

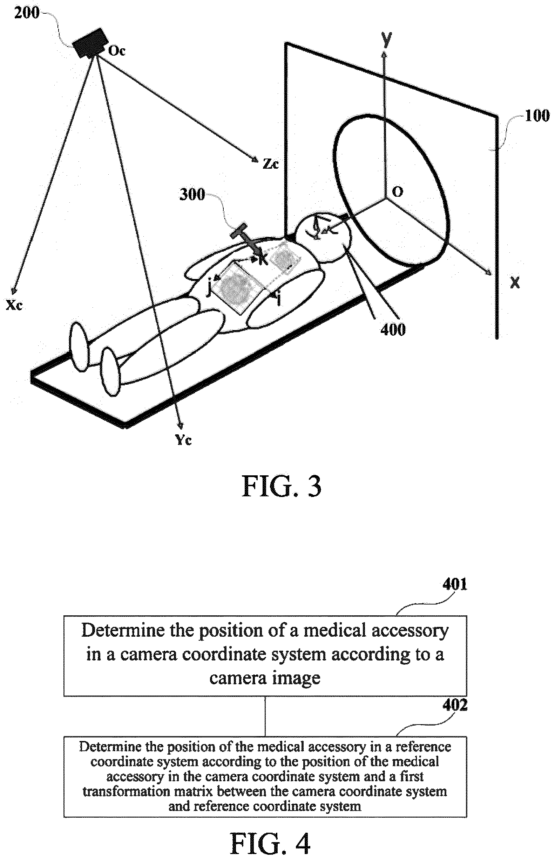

In some embodiments, the medical imaging method may be performed in a medical imaging system. FIG. 3 is a schematic diagram of a medical imaging system according the disclosure. The medical imaging system may include a processor (not shown in the figure) and a display (not shown in the figure). The processor may determine the position of a medical accessory in a reference coordinate system based on a camera image acquired by an auxiliary camera, and reconstruct a medical image according to a scanning result and based on the reference coordinate system. The display may display in real time the position of the medical accessory in the medical image. As shown in FIG. 3, the medical imaging system may further include a CT imaging device 100 and an auxiliary camera 200. The CT imaging device 100 may be configured to obtain a scanning result, and the structure of the CT imaging device may refer to the foregoing content, which will not be further described here. The auxiliary camera 200 may be used to acquire a camera image. The present application is not limited to this, and the medical imaging system may further include other medical imaging devices as described above.

In some embodiments, the auxiliary camera 200 may be various components having a camera function. For example, the auxiliary camera may be a depth camera, a thermal imaging camera or the like. The auxiliary camera 200 may capture an image of a subject (for example, the medical accessory 300, the subject 400, or the like) appearing within a camera range of the auxiliary camera 200, and generate a camera image including the subject based on a camera coordinate system (Xc, Yc, Zc).

In some embodiments, the origin Oc of the camera coordinate system (Xc, Yc, Zc) may be located at the optical center of the auxiliary camera, the Zc axis may be parallel to an optical axis of the auxiliary camera, and the Xc axis and the Yc axis perpendicular to each other and each perpendicular to the Zc axis. The present application is not limited to this, and the camera coordinate system may be configured in other forms.

In some embodiments, in 201, the position of the medical accessory in the camera coordinate system may be determined based on the camera image, so that the position of the medical accessory in the reference coordinate system can be determined by performing coordinate transformation on the position of the medical accessory in the camera coordinate system. FIG. 4 is a schematic diagram of an example of 201 according to an embodiment of the present application. As shown in FIG. 4, 201 may include:
401: Determining the position of the medical accessory in a camera coordinate system according to the camera image; and
402: Determining the position of the medical accessory in the reference coordinate system according to the position of the medical accessory in the camera coordinate system and a first transformation matrix between the camera coordinate system and reference coordinate system.

In some embodiments, the medical accessory 300 may be various components used in interventional procedures. For example, the medical accessory may be a puncture needle or puncture needle assembly, a guide wire or guide wire assembly, a catheter or catheter assembly, or the like. The number of medical accessories may be one or more.

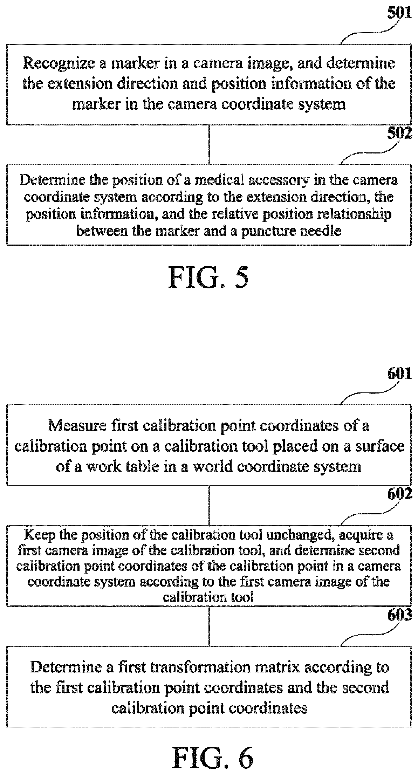

Using the medical accessory 300 being a puncture needle assembly as an example, the medical accessory 300 may include a puncture needle and a marker. In 401, the position of the marker in the camera image may be determined, thereby determining the position of the puncture needle in the camera image. FIG. 5 is a schematic diagram of an example of 401 according to an embodiment of the present application. As shown in FIG. 5, 401 may include:
501: Recognizing the marker in the camera image, and determining the extension direction and position information of the marker in the camera coordinate system; and
502: Determining the position of the medical accessory in the camera coordinate system according to the extension direction, the position information, and the relative positional relationship between the marker and the puncture needle.

In 501, the specific manner of recognizing the marker in the camera image and determining the extension direction and the position information of the marker in the camera coordinate system may refer to the related art, which will not be further described here.

In some embodiments, the relative positional relationship between the marker and the puncture needle (for example, the included angle between the extension directions of the marker and the puncture needle and/or the distance between the marker and the puncture needle) may be prestored. Therefore, after the extension direction and the position information of the marker are acquired, the extension direction and the position information of the puncture needle can be determined according to the known relative positional relationship, and thus the position of the medical accessory in the camera coordinate system can be determined.

In some embodiments, an included angle may be preset between the extension direction of the marker and the extension direction of the puncture needle. For example, the marker may be parallel or perpendicular to the puncture needle, etc.

In some embodiments, the marker may be fixed at a preset position of the puncture needle, so the distance between the marker and each position of the puncture needle (for example, a needle tip) can be predetermined.

The present application is not limited to this, and the marker may also carry information related to the medical accessory. For example, the marker may be a figure capable of carrying information. By recognizing the marker, information indicating the relative positional relationship between the marker and the puncture needle can be acquired. Therefore, it is not necessary to prestore the relative positional relationship.

In some embodiments, the method may include at least one of the following: a component having a preset temperature, a component having a preset shape and/or pattern, or a component emitting light according to a preset rule. The marker can be recognized accurately and reliably in the camera image by configuring the marker as the above form. The present application is not limited to this, and the marker may also be in other forms.

In some embodiments, the marker may be a cannula sleeved on the peripheral side of the puncture needle. The marker is configured in the above manner, so that the extension direction of the marker can be ensured to be parallel to the extension direction of the puncture needle, thereby facilitating determination of the extension direction and the position information of the puncture needle according to the extension direction and the position information of the marker. The present application is not limited to this, and the marker may also be in other forms.

In some embodiments, the cannula may be a separate reusable accessory. The present application is not limited to this, and the cannula may be a component that is integrally formed with the puncture needle.

FIG. 5 is used as an example to describe the implementation of 401 in the embodiment of the present application. The present application is not limited to this, and the position of the medical accessory in the camera coordinate system may be determined in other manners. For example, the medical accessory and the position thereof are detected using a deep learning algorithm, and according to camera depth and RGB data.

In some embodiments, the reference coordinate system may include a world coordinate system (X, Y, Z). The present application is not limited to this, and the reference coordinate system may further include other coordinate systems.

In some embodiments, as shown in FIG. 3, the origin O of the world coordinate system (X, Y, Z) may be located at the center of the scanning gantry (for example, the scanning gantry of the CT imaging device 100). By configuring the origin O of the world coordinate system (X, Y, Z) at the center of the scanning gantry, the processing of reconstructing the medical image described hereinafter can be simplified, and the quantity of calculations for coordinate transformation can be reduced. The present application is not limited to this, and the origin O of the world coordinate system (X, Y, Z) may also be located at other positions.

In some embodiments, one coordinate axis (for example, the X axis) of the world coordinate system (X, Y, Z) may be parallel to a short side of a patient table 102, one coordinate axis (for example, the Z axis) may be parallel to a long side of the patient table 102, and one coordinate axis (for example, the Y axis) may be perpendicular to the patient table 102. By configuring the coordinate axes of the world coordinate system as such, the processing of reconstructing the medical image described hereinafter can be further simplified, and the calculation quantity for coordinate transformation can be reduced. The present application is not limited to this, and the three coordinate axes of the world coordinate system (X, Y, Z) may also be configured in other manners.

In some embodiments, there may be various transformation relationships between the camera coordinate system (Xc, Yc, Zc) of the auxiliary camera and the world coordinate system (X, Y, Z), for example, affine transformation, which transforms a point (or position) in the camera coordinate system into a point (or position) in the world coordinate system by means of rotation and/or scaling and/or translation.

In some embodiments, the transformation relationship between the camera coordinate system (Xc, Yc, Zc) of the auxiliary camera and the world coordinate system (X, Y, Z) may be represented by means of a first transformation matrix. The first transformation matrix may be determined in various manners. FIG. 6 is a schematic diagram of a method for determining a first transformation matrix. As shown in FIG. 6, the method for determining a first transformation matrix may include:
601: Measuring first calibration point coordinates of a calibration point on a calibration tool placed on a surface of a work table (for example, the patient table 102 or the like) in a world coordinate system;
602: Keeping the position of the calibration tool unchanged, acquiring a first camera image of the calibration tool, and determining second calibration point coordinates of the calibration point in the camera coordinate system according to the first camera image of the calibration tool; and
603: Determining a first transformation matrix according to the first calibration point coordinates and the second calibration point coordinates.

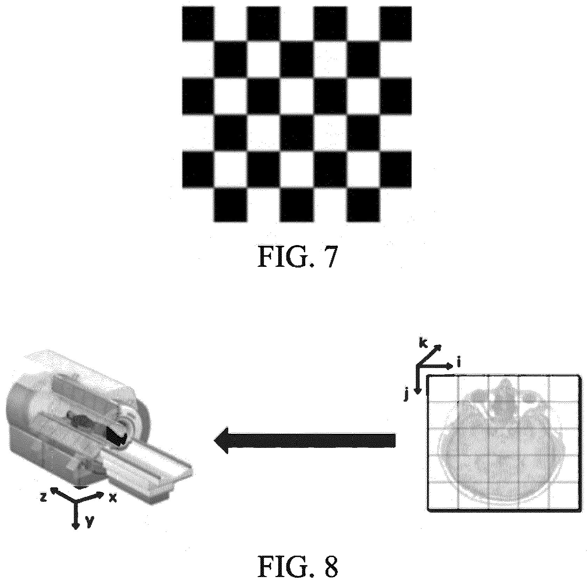

In some embodiments, the calibration tool may be in various forms. FIG. 7 is a schematic diagram of a calibration tool according to an embodiment of the present application. As shown in FIG. 7, the calibration tool may be a flat plate having a black-and-white pattern, and a point connected by each black-and-white grid may serve as a calibration point.

In some embodiments, the CT imaging device 100 may scan a subject to be scanned to generate a scanning result. The scanning result may be represented in various forms. For example, the scanning result may be represented by means of digital imaging and communications in medicine (DICOM) data or the like, thereby facilitating data processing and exchange.

In some embodiments, the scanning result may include a plurality of voxels, and the position of each of the voxels may be represented by coordinates (i, j, k) in the scanning coordinate system (I, J, K). For example, the scanning result may include a plurality of sections, the coordinate k may represent the section in which the voxel is located, the coordinate i may represent a column in which the voxel is located in the section, and the coordinate j may represent a row in which the voxel is located in the section. The present application is not limited to this, and the coordinates (i, j, k) may further include other meanings.

In some embodiments, in 202, the medical image is reconstructed according to the scanning result. FIG. 8 is a schematic diagram of a scanning coordinate system (I, J, K) and a world coordinate system (X, Y, Z) according to an embodiment of the present application. As shown in FIG. 8, in 202, coordinate transformation may be performed on the position of the scanning result in the scanning coordinate system so as to obtain the position of the scanning result in the reference coordinate system, that is, the medical image is reconstructed in the reference coordinate system.

In some embodiments, there may be various transformation relationships between the scanning coordinate system (I, J, K) and the world coordinate system (X, Y, Z), for example, affine transformation, which transforms a pixel in the scanning coordinate system (I, J, K) into a point (or position) in the world coordinate system (X, Y, Z) by means of rotation and/or scaling and/or translation.

In some embodiments, the transformation relationship between the scanning coordinate system (I, J, K) and the world coordinate system (X, Y, Z) may be represented by a second transformation matrix. Parameters in the second transformation matrix may be obtained from the scanning result (for example, DICOM data). Thus, each voxel can be mapped into the world coordinate system according to the scanning result (for example, DICOM data).

For example, using a pixel (i, j, 0) in the scanning coordinate system as an example, the pixel may be transformed into coordinates (*Pₓ*, *P_{y}*, *P_{z}*) in the world coordinate system according to the second transformation matrix M. $\left⌊\begin{matrix}{P}_{x} \\ {P}_{y} \\ {P}_{z} \\ 1\end{matrix}\right⌋ = \left⌊\begin{matrix}{X}_{x} {Δ}_{i} & {Y}_{x} {Δ}_{j} & 0 & {S}_{x} \\ {X}_{y} {Δ}_{i} & {Y}_{y} {Δ}_{j} & 0 & {S}_{y} \\ {X}_{z} {Δ}_{i} & {Y}_{z} {Δ}_{j} & 0 & {S}_{z} \\ 0 & 0 & 0 & 1\end{matrix}\right⌋ \left[\begin{matrix}i \\ j \\ 0 \\ 1\end{matrix}\right] = M \left[\begin{matrix}i \\ j \\ 0 \\ 1\end{matrix}\right]$ (Formula 1)
where (*Pₓ, P_{y}, P_{z}*) is the world coordinates of the voxel (i, j) in an image plane in millimeters (mm); (*S_{y}, S_{y}, S_{z}*) is the image position (subject) relative to the origin O in mm; (*Xₓ, X_{y}, X_{z}*) is a row (X) direction cosine value of an image direction (subject); (*Yₓ, Y_{y}, Y_{z}*) is a column (Y) direction cosine value of the image direction (subject); i is a column index of the image plane, and the index of the first column is, for example, 0; Δᵢ is the column pixel resolution of the pixel pitch in mm; j is a row index of the image plane, and the index of the first row is, for example, 0; and Δⱼ is the line pixel resolution of the pixel pitch in mm. Parameters (for example, (*Sₓ, S_{y}, S_{z}*), (*Xₓ, X_{y}, X_{z}*), (*Yₓ, Y_{y}, Y_{z}*), Δᵢ and Δⱼ) in the second transformation matrix M may be acquired from DICOM data. For a specific determination method, reference may made to the related art, which will not be further described here.

In some embodiments, the position of the subject when the scanning result is generated may be different from the position of the subject during the interventional procedure. For example, the subject may be located within a scanning gantry (for example, a first position) when being scanned; and the subject may be moved out of the scanning gantry (for example, a second position) when being subjected to the interventional procedure. For example, the subject is kept on the patient table 102, and the subject is moved from the patient table 102 to the first position during scanning; and after the scanning ends, the subject is moved from the patient table 102 to the second position, so that the subject is subjected to the interventional procedure at the second position.

In 202, when the subject is moved from the first position in which scanning is performed to the second position, the medical image may be also reconstructed according to the relative positional relationship between the first position and the second position. For example, after coordinate transformation is performed on the position of the scanning result in the scanning coordinate system according to the second transformation matrix, the transformed coordinates are transformed again according to said relative positional relationship, so as to obtain the position of the scanning result in the reference coordinate system.

The relative positional relationship between the first position and the second position may be determined according to the displacement of the patient table 102. The present application is not limited to this, and the relative positional relationship between the first position and the second position may also be determined in other manners. For example, at least three markers are arranged on the surface of the subject, and the relative positional relationship between the first position and the second position is determined according to the positions of the three markers when the subject is scanned and the positions of the three markers when the interventional procedure is performed. As a result, even if the subject is displaced relative to the patient table 102, said relative positional relationship may be accurately determined.

In some embodiments, in 203, when the position of the medical accessory in the reference coordinate system and the position of the medical image in the reference coordinate system are determined, the position of the medical accessory in the medical image may be displayed in the reference coordinate system in real time according to said position information. For the specific manner of displaying according to said position reference may be made to the related art, which will not be further described here.

In some embodiments, the position of the medical accessory may be the position of at least one part of the medical accessory. Using the medical accessory comprising a puncture needle as an example, the position may be the position of a needle tip of the puncture needle, or the position may be the position of the whole puncture needle, or the like.

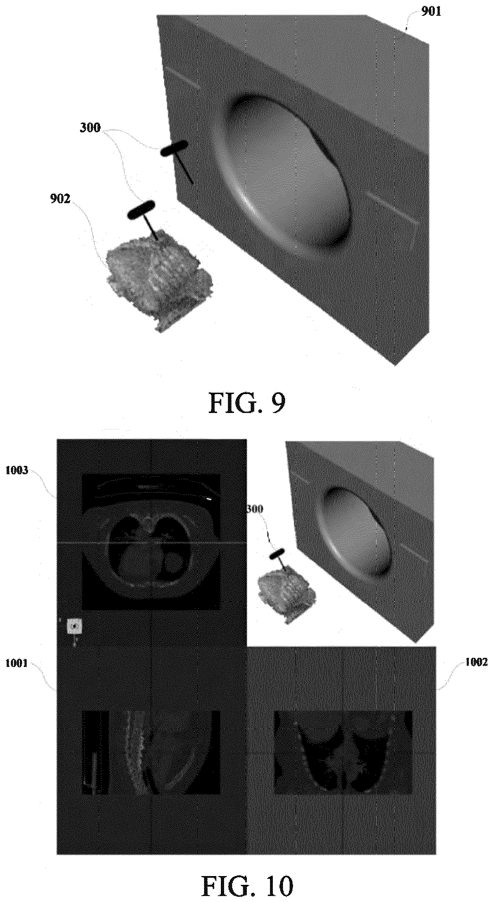

In some embodiments, the medical image in the reference coordinate system may comprise a three-dimensional image, wherein the three-dimensional image, for example, is generated according to the scanning result (for example, DICOM data) by means of a three-dimensional modeling algorithm. FIG. 9 is a schematic diagram of a displayed image according to an embodiment of the present application. As shown in FIG. 9, in 203, the position of the medical accessory 300 may be displayed in real time in a three-dimensional medical image 901. The medical image 901 may include an area 902 corresponding to the scanning result, and thus the relative positional relationship between the medical accessory 300 and the area 902 can be displayed in real time. Thus, the operator promptly adjusting the angle and position of the medical accessory 300 according to the relative positional relationship is facilitated.

In some embodiments, other auxiliary information may also be displayed in the three-dimensional image of the medical image. For example, a planned movement path of the medical accessory, and/or information indicating the relative positional relationship between the medical accessory and a starting point and/or a target point of the movement path, and/or a predicted path determined according to the position and the extension direction of the medical accessory, and/or the camera image are subjected to superimposed display in the three-dimensional image.

For example, after the scanning result is acquired, a movement path of the medical accessory may be planned according to the scanning result, and it is desirable to move the medical accessory according to the planned movement path during the interventional procedure, so that the safety and reliability of the interventional procedure can be ensured. The movement path may include a starting point, a target point, and a path between the starting point and the target point. The starting point may be, e.g., a needle entry point of the puncture needle, and the target point may be, e.g., the target position at which the puncture needle is to arrive, for example, a lesion position, or the like. By performing superimposed display of the movement path in the three-dimensional image, the operator can visually understand whether the current position of the medical accessory has deviated from the movement path, so that the angle and the position of the medical accessory 300 can be promptly adjusted.

Alternatively, information indicating the relative positional relationship between the medical accessory and the starting point and/or the target point of the movement path may be subjected to superimposed display. The information, for example, may include angle information and/or distance information of the needle tip of the puncture needle relative to the starting point and/or the target point, or may include an icon or the like directed from the needle tip of the puncture needle to the starting point and/or the target point, thereby facilitating the operator more directly understanding the position of the medical accessory.

Alternatively, a predicted path determined according to the position and the extension direction of the medical accessory may also be subjected to superimposed display. For example, the predicted path is represented by means of lines of different colors or different shapes from those of the icon of the medical accessory, thus helping the operator to understand the position at which the medical accessory is about to arrive when the medical accessory is pushed according to the current position and angle.

Alternatively, the camera image acquired by the auxiliary camera may be subjected to superimposed display. The camera image acquired by the auxiliary camera may include a subject, and the camera image is subjected to superimposed display in the three-dimensional image, thereby facilitating the operator knowing the relative positional relationship among the medical accessory, the area 902 and the subject.

In some embodiments, the medical image may further include a two-dimensional image displayed on one or a plurality of planes. The two-dimensional image may be generated according to the scanning result (for example, DICOM data) by means of a multiplanar reconstruction algorithm. For example, the two-dimensional image may include a sagittal plane, a coronal plane and a cross section. The present application is not limited to this, and the two-dimensional image may further include a planar image in other directions.

In some embodiments, in addition to displaying in real time the position of the medical accessory in the medical image, a two-dimensional image corresponding to the position of the medical accessory may also be displayed in real time according to the position of the medical accessory, thereby facilitating the operator more clearly understanding the position of the medical accessory in the medical image.

In some embodiments, the two-dimensional image corresponding to the position of the medical accessory may include: a first two-dimensional image that passes through the position of the medical accessory, and/or a second two-dimensional image that passes through the target point of the medical accessory, and that is perpendicular to the first two-dimensional image passing through the position of the medical accessory. Thus, the first two-dimensional image can provide the operator with information related to the tissue and/or structure near the position of the medical accessory, and the second two-dimensional image can provide the operator with information related to the tissue and/or structure near the position of the lesion, thereby facilitating the operator understanding the positions of the medical accessory and the target point.

In some embodiments, the two-dimensional image corresponding to the position of the medical accessory may further include: a first two-dimensional image that passes through the position of the medical accessory, and a third two-dimensional image that passes through the position of the medical accessory, and that is perpendicular to the first two-dimensional image. Therefore, more detailed information related to the tissue and/or structure near the position of the medical accessory can be provided to the operator.

FIG. 10 is another schematic diagram of a displayed image according to an embodiment of the present application. As shown in FIG. 10, a sagittal plane 1001, a coronal plane 1002, and a cross section 1003 passing through the position of the needle tip of the puncture needle may be displayed according to the position of the medical accessory 300, for example, the position of the needle tip of the puncture needle.

In some embodiments, the first two-dimensional image and/or the second two-dimensional image and/or the third two-dimensional image may include marking information of the target point. The marking information may include information indicating the position of the target point, and/or information related to the relative positional relationship between the medical accessory and the target point. For example, the marking information may include the direction and the distance in which the medical accessory is required to be moved from the current position to the target point. Thus, the operator accurately adjusting the position and angle of the medical accessory is facilitated.

As shown in FIG. 2, the method further includes:
204: Generating a grid matrix according to the scanning result, wherein a starting point and/or a target point of a planned movement path of the medical accessory is marked in the grid matrix; and
205: Superimposing the grid matrix in the camera image.

Thus, the position information of the starting point and/or the target point can be provided to the operator in the camera image, which helps the operator to visually understand the position of the starting point and/or the target point from the camera image. Furthermore, the grid matrix can be generated without external physical assemblies, and thus the procedure can be simplified.

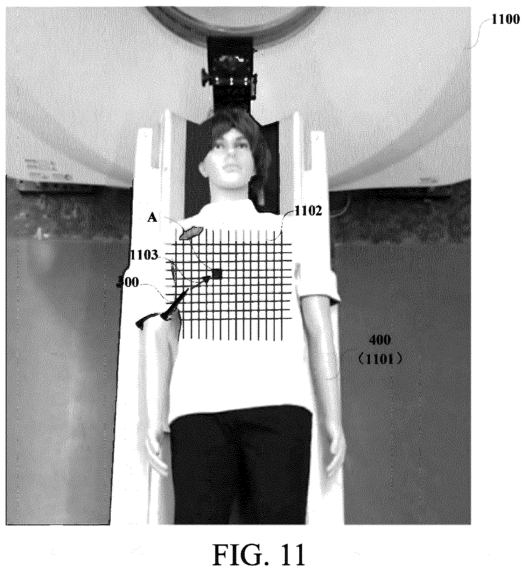

FIG. 11 is another schematic diagram of a displayed image according to an embodiment of the present application. As shown in FIG. 11, a camera image 1100 may include a real image 1101 of a subject 400, a grid matrix 1102 being superimposed on the camera image 1100, and a starting point A being marked on the grid matrix 1102, thereby enabling an operator to visually understand the position of the starting point A on the subject 400, thus facilitating the operator quickly positioning the medical accessory 300 at the starting point A, and improving the efficiency and accuracy of the interventional procedure.

Conventionally, in order to facilitate the operator determining the starting point A, it is generally necessary to attach a metal mesh to a surface of the subject 400, scan the subject 400 to which the metal mesh is attached, and acquire a scanning result. The position of the starting point A on the subject 400 is determined according to relative position information of the metal mesh image in the scanning result and the starting point A of the planned movement path. For example, according to the scanning result, it is determined that the starting point A is located in the grid in the n^{th} row and the m^{th} column of the metal mesh, and the operator finds the grid in the n^{th} row and the m^{th} column on the metal mesh attached to the surface of the subject 400, thereby determining the position of the starting point A on the subject 400.

Compared with the prior art, by superimposing a grid matrix in the camera image, no additional disposable auxiliary consumables (for example, the metal mesh) are required, no additional hardware is required to assist the positioning of the medical accessory, and the costs of the interventional scan can be reduced.

In some embodiments, the grid matrix 1102 may be generated from the scanning result. Using DICOM data as an example, each pixel in the DICOM data can be mapped from the scanning coordinate system to the world coordinate system, so that each pixel can be mapped into the camera coordinate system. As a result, the starting point and/or the target point of the planned path in the scanning coordinate system can be mapped into the camera coordinate system, and a mesh matrix marked with the starting point and/or the target point can be generated.

In some embodiments, the position of the medical accessory and/or information indicating the relative positional relationship between the medical accessory and the starting point and/or the target point may be displayed on the grid matrix in real time. As shown in FIG. 11, the image of the medical accessory 300 may be displayed in the camera image 1100, so the position of the medical accessory 300 relative to the starting point A can be more visually provided to the operator, and the operator can visually understand the distance between the current position of the medical accessory 300 and the starting point A using the grid matrix 1102 as a scale.

As shown in FIG. 11, the information 1103 indicating the relative positional relationship between the medical accessory 300 and the starting point and/or the target point may be an arrow pointing from the current position of the medical accessory 300 to the starting point and/or the target point, or distance information, thereby facilitating the operator to quickly place the medical accessory 300 at the correct position.

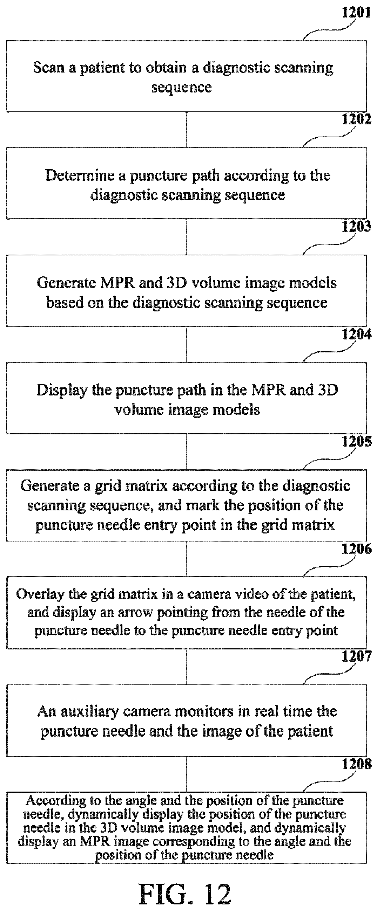

FIG. 12 is another schematic diagram of a medical image imaging method according to an embodiment of the present application. Referring to FIG. 12, a medical imaging method according to the embodiment of the present application is exemplarily described below using the subject being a patient and the medical accessory comprising a puncture needle assembly as an example. As shown in FIG. 12, the medical image imaging method may include:
1201: Scanning the patient to obtain a diagnostic scanning sequence;
1202: Determining a puncture path according to the diagnostic scanning sequence;
1203: Generating multiplanar reconstruction (MPR) and 3D volume image models based on the diagnostic scanning sequence;
1204: Displaying the puncture path in the MPR and 3D volume image models;
1205: Generating a grid matrix according to the diagnostic scanning sequence, and marking the position of the puncture needle entry point in the grid matrix;
1206: Overlaying the grid matrix in a camera video of the patient, and displaying an arrow pointing from the needle of the puncture needle to the puncture needle entry point;
1207: Monitoring in real time, by the auxiliary camera, the image of the puncture needle and the patient, so that a user can place the puncture needle at the needle entry point according to the grid of the grid matrix in the camera video; and
1208: According to the angle and the position of the puncture needle, dynamically displaying the position of the puncture needle in the 3D volume image model, and dynamically displaying an MPR image corresponding to the angle and the position of the puncture needle, so that the user can move the puncture needle to the target point to complete the puncture.

It should be noted that FIG. 2, FIG. 4, FIG. 5, FIG. 6 and FIG. 12 merely schematically illustrate the embodiments of the present application, but the present application is not limited to this. For example, the order of execution between operations may be appropriately adjusted. In addition, some other operations may be added or some operations may be omitted. Those skilled in the art can make appropriate modifications according to the above content, rather than being limited by the descriptions of FIG. 2, FIG. 4, FIG. 5, FIG. 6 and FIG. 12.

As can be seen from the above embodiment, the position of the medical accessory in the reference coordinate system is determined based on the camera image acquired by the auxiliary camera, and the medical image is reconstructed according to the scanning result and based on the reference coordinate system. Therefore, the position of the medical accessory in the medical image can be displayed in real time, and thus the convenience of the procedure can be improved, the positioning process of the medical accessory can be simplified and shortened, and the operator can quickly and accurately move the medical accessory to the target position.

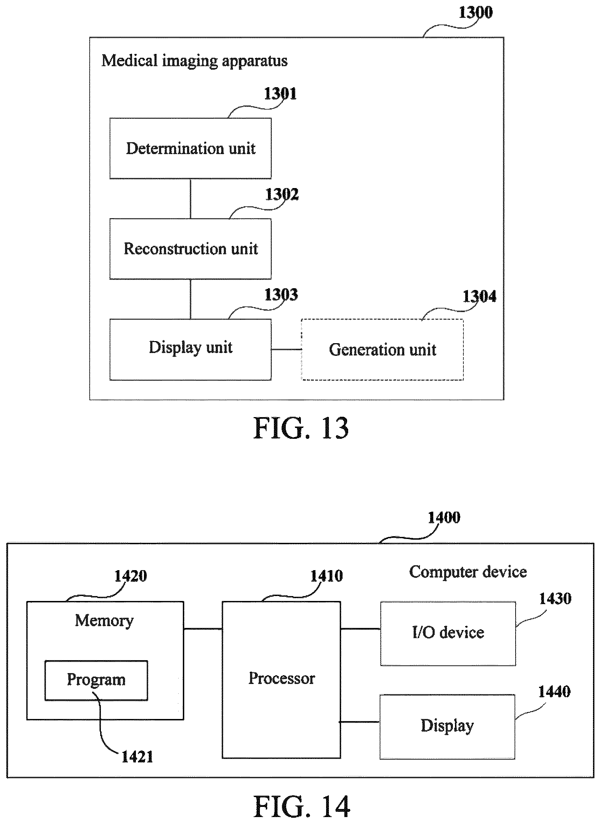

The present disclosure also describes a medical imaging device that serves as an illustrative example useful for understanding the invention to which this European patent relates and repetitive content from the preceding embodiments is not given here. FIG. 13 is a schematic diagram of a medical imaging apparatus of an embodiment of the present application. As shown in FIG. 13, the medical imaging apparatus 1300 includes:

A determination unit 1301, which determines the position of a medical accessory in a reference coordinate system based on a camera image acquired by an auxiliary camera;
A reconstruction unit 1302, which reconstructs a medical image according to a scanning result and based on the reference coordinate system; and
A display unit 1303, which displays in real time the position of the medical accessory in the medical image.

In some embodiments, the reference coordinate system includes a world coordinate system, and the origin of the world coordinate system is located at the center of a CT gantry.

In some embodiments, the medical imaging apparatus may further include: a generation unit 1304, which generates a grid matrix according to the scanning result, wherein a starting point and/or a target point of a planned movement path of the medical accessory are/is marked in the grid matrix. The display unit 1303 superimposes the grid matrix on the camera image.

In some embodiments, the display unit 1303 displays in real time the position of the medical accessory and/or information indicating the relative positional relationship between the medical accessory and the starting point and/or the target point on the grid matrix.

In some embodiments, the medical image includes a three-dimensional image and/or a two-dimensional image displayed on one or a plurality of planes.

In some embodiments, the display unit 1303 displays in real time, according to the position of the medical accessory, the two-dimensional image corresponding to the position of the medical accessory.

In some embodiments, the two-dimensional image corresponding to the position of the medical accessory includes: a first two-dimensional image that passes through the position of the medical accessory, and/or a second two-dimensional image that passes through the target point of the medical accessory and that is perpendicular to the first two-dimensional image passing through the position of the medical accessory.

In some embodiments, the two-dimensional image includes marking information of the target point.

In some embodiments, the marking information includes information related to the relative positional relationship between the medical accessory and the target point.

In some embodiments, the display unit 1303 performs superimposed display in a three-dimensional image of the medical image on a planned movement path of the medical accessory, and/or information indicating the relative positional relationship between the medical accessory and a starting point and/or a target point of the movement path, and/or a predicted path determined according to the position and the extension direction of the medical accessory, and/or the camera image.

In some embodiments, the determination unit 1301 determines the position of the medical accessory in a camera coordinate system according to the camera image, and determines the position of the medical accessory in the reference coordinate system according to the position of the medical accessory in the camera coordinate system and a first transformation matrix between the camera coordinate system and the reference coordinate system.

In some embodiments, the medical accessory includes a puncture needle and a marker; the determination unit 1301 recognizes the marker in the camera image, and determines the extension direction and position information of the marker in the camera coordinate system; and the position of the medical accessory camera in the camera coordinate system is determined according to the extension direction, the position information, and the relative positional relationship between the marker and the puncture needle.

In some embodiments, a preset included angle is formed between the extension direction of the marker and the extension direction of the puncture needle, and/or the marker is fixed at the preset position of the puncture needle; and/or the marker carries information related to the medical accessory.

In some embodiments, the marker is a cannula sleeved on the peripheral side of the puncture needle.

In some embodiments, the marker includes at least one of the following: a component having a preset temperature, a component having a preset shape and/or pattern, or a component emitting light according to a preset rule.

In some embodiments, the reconstruction unit 1302 reconstructs the medical image in the reference coordinate system according to the position of the scanning result in the scanning coordinate system and a second transformation matrix between the scanning coordinate system and the reference coordinate system.

In some embodiments, when a scanned subject moves from a first position in which CT scanning is performed to a second position, the reconstruction unit 1302 also reconstructs the medical image according to the relative positional relationship between the first position and the second position.

As can be seen from the above embodiment, the position of the medical accessory in the reference coordinate system is determined based on the camera image acquired by the auxiliary camera, and the medical image is reconstructed according to the scanning result and based on the reference coordinate system. Therefore, the position of the medical accessory in the medical image can be displayed in real time, so that the convenience of the procedure can be improved, the positioning process of the medical accessory can be simplified and shortened, and the operator can quickly and accurately move the medical accessory to the target position.

Further provided in the embodiments of the present application is a computer device useful for understanding the invention. FIG. 14 is a schematic diagram of a computer device according to an embodiment of the present application. As shown in FIG. 14, the computer device 1400 may include: one or a plurality of processors (for example, a central processing unit (CPU)) 1410 and one or a plurality of memories 1420. The memory 1420 is coupled to the processor 1410. The memory 1420 may store a program 1421 (for example, a medical imaging program or the like) and/or data (for example, a camera image, a scanning result or the like). The program 1421 is executed under the control of the processor 1410. The memory 1420 may include, for example, a ROM, a floppy disk, a hard disk, an optical disk, a magneto-optical disk, a CD-ROM, or a non-volatile memory card.

In some embodiments, the functions of the medical imaging apparatus 1300 are integrated into the processor 1410 for implementation. The processor 1410 is configured to implement the medical imaging method as described in the preceding embodiments. For the implementation of the processor 1410, reference may be made to the aforementioned embodiments, which will not be described again here.

In some embodiments, the medical imaging apparatus 1300 and the processor 1410 are configured separately. For example, the medical imaging apparatus 1300 may be configured as a chip connected to the processor 1410, and the functions of the medical imaging apparatus 1300 can be achieved by means of the control of the processor 1410.

Furthermore, as shown in FIG. 14, the computer device 1400 may further include: an input device 1430 and a display 1440. The functions of said components are similar to those in the prior art, and are not described again here. It should be noted that the computer device 1400 does not necessarily include all of the components shown in FIG. 14. In addition, the computer device 1400 may further include components not shown in FIG. 14, for which reference may be made to the related art.

The processor 1410 may communicate with a medical device, the display or the like in response to an operation of the input device, and may also control input actions and/or the state of the input device. The processor 1410 may also be referred to as a microcontroller unit (MCU), a microprocessor or a microcontroller or other processor apparatuses and/or logic apparatuses. The processor 1410 may include a reset circuit, a clock circuit, a chip, a microcontroller and the like. The functions of the processor 1410 may be integrated on a main board of the medical device (for example, the processor 1410 is configured as a chip connected to a main board processor (CPU)), or may be configured independently of the main board, and the embodiments of the present application are not limited to this.

In some embodiments, the computer device may be a computer server or a cloud platform or workstation, etc., and the embodiments of the present application are not limited thereto.

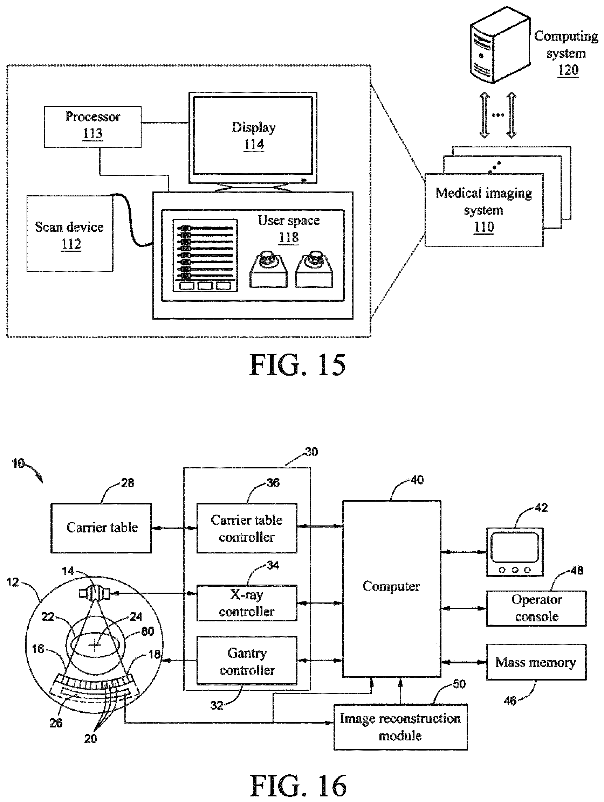

The present disclosure also describes a medical imaging system that serves as an illustrative example useful for understanding the invention to which this European patent relates. FIG. 15 is another schematic diagram of a medical imaging system according to an embodiment of the present application. As shown in FIG. 15, the medical imaging system 110 includes suitable hardware, software, or a combination thereof for supporting medical imaging. For example, the medical imaging system 110 may be a CT system configured to generate and/or render a CT image, or an ultrasound system configured to generate and/or render an ultrasound image, or a magnetic resonance system configured to generate and/or render a magnetic resonance image, or the like. As shown in FIG. 15, the medical imaging system 110 may include a processor 113 and a display 114. As described above, the processor 113 may determine the position of a medical accessory in a reference coordinate system based on a camera image acquired by an auxiliary camera, and reconstruct a medical image according to a scanning result and based on the reference coordinate system. The display 114 may display in real time the position of the medical accessory in the medical image. The specific implementations are as described above, and will not be described again here.

The display 114 may be configured to display images (e.g., via a screen). In some cases, the display 114 may also be configured to at least partially generate the displayed image. In addition, the display 114 may further support user input/output. For example, in addition to images, the display 114 may further provide (e.g., via the screen) user feedback (e.g., information related to the system, the functions and settings thereof, etc.). The display 114 may further support user input (e.g., via user controls 118) to, for example, allow control of medical imaging. User input can involve controlling the display of images, selecting settings, specifying user preferences, requesting feedback, etc.

As shown in FIG. 15, the medical imaging system 110 may further include a scan device 112. The scan device may be fixed or movable. The scan device 112 may be configured to generate and/or capture specific types of imaging signals (and/or data corresponding thereto) by means such as moving over a subject to be examined (or a portion thereof), and may include suitable circuits for performing and/or supporting such functions. The scan device 112 may be an ultrasonic probe, an MRI scanner, a CT scanner, or any suitable imaging device.

In some embodiments, the medical imaging system 110 may further include a camera device (not shown in the figure). The camera device (for example, the aforementioned auxiliary camera) may acquire images of subjects (for example, the medical accessory and the subject) located within the camera range thereof.

In some embodiments, the medical imaging system 110 may further incorporate additional and dedicated computing resources, such as one or more computing systems 120. In this regard, each computing system 120 may include circuits, interfaces, logic, and/or code suitable for processing, storing, and/or communicating data. The computing system 120 may be a specialized device configured for use specifically in conjunction with medical imaging, or it may be a general-purpose computing system (e.g., a personal computer, server, etc.) that is set up and/or configured to perform the operations described below with respect to computing system 120. The computing system 120 may be configured to support the operation of the medical imaging system 110, as described below. In this regard, various functions and/or operations can be offloaded from the imaging system, which may simplify and/or centralize certain aspects of processing to reduce costs (by eliminating the need to add processing resources to the imaging system).

The computing system 120 may be set up and/or arranged for use in different ways. For example, in some specific implementations, a single computing system 120 may be used; and in other specific implementations, a plurality of computing systems 120 are configured to work together (for example, configured based on distributed processing), or individually. Each of the computing systems 120 is configured to process a specific aspect and/or function, and/or to process data only for a specific medical imaging system 110.

In some embodiments, the computing system 120 may be local (for example, co-located with one or a plurality of medical imaging systems 110, such as within the same facility and/or the same local network); and in other specific embodiments, the computing system 120 may be remote, and thus accessible only by means of a remote connection (for example, by means of the Internet or other available remote access technologies). In particular specific implementations, the computing system 120 may be configured in a cloud-based manner and may be accessed and/or used in a substantially similar manner to accessing and using other cloud-based systems.

Once the data is generated and/or configured in the computing system 120, the data can be copied and/or loaded into the medical imaging system 110. For example, data may be loaded via a directed connection or link between the medical imaging system 110 and the computing system 120. In this regard, communication between the different components of the setup can be performed using available wired and/or wireless connections and/or according to any suitable communication (and/or networking) standards or protocols. Optionally or additionally, the data may be loaded indirectly into the medical imaging system 110. For example, data may be stored in a suitable machine-readable medium (for example, a flash memory card or the like) and then loaded into the medical imaging system 110 using the machine-readable medium (on-site, for example, by a user of the system (such as an imaging clinician) or authorized personnel); or the data may be downloaded to a locally communicative electronic device (for example, a laptop or the like) and then said electronic device used on-site (for example, by a user of the system or authorized personnel) to upload the data to the medical imaging system 110 by means of a direct connection (for example, a USB connector or the like).

In operation, the medical imaging system 110 may be used to generate and present (for example, render or display) images during a medical examination or treatment and/or used in conjunction therewith to support user input/output. The images can be 2D, 3D, and/or 4D images. The particular operations or functions performed in the medical imaging system 110 to facilitate the generation and/or presentation of images depend on the type of system (i.e., the means used to obtain and/or generate the data corresponding to the images).

An exemplary description is performed below by using the medical imaging system being a CT system as an example. FIG. 16 is a schematic diagram of a CT system 10 according to an embodiment of the present application. As shown in FIG. 16, the system 10 includes a gantry 12. An X-ray source 14 and a detector 18 are arranged opposite to each other on the gantry 12. The detector 18 is composed of a plurality of detector modules 20 and a data acquisition system (DAS) 26. The DAS 26 is configured to convert sampled analog data of analog attenuation data received by the plurality of detector modules 20 into digital signals for subsequent processing. The system 10 may further include a camera device (not shown in the figure) for acquiring images of the medical accessory.

In some embodiments, the system 10 is used to acquire, from different angles, projection data of a subject to be examined. Thus, components on the gantry 12 are used to rotate around a rotation center 24 to acquire projection data. During rotation, the X-ray radiation source 14 is configured to emit toward the detector 18 X-rays 16 that penetrate the subject to be examined. Attenuated X-ray beam data is preprocessed and then used as projection data of a target volume of the subject. An image of the subject to be examined may be reconstructed on the basis of the projection data. The reconstructed image may display internal features of the subject to be examined. These features include, for example, the lesion, size, and shape of body tissue structure. The rotation center 24 of the gantry also defines the center of a scanning field 80.

In some embodiments, the system 10 includes a control mechanism 30. The control mechanism 30 may include an X-ray controller 34 used to provide power and timing signals to the X-ray radiation source 14. The control mechanism 30 may further include a gantry controller 32 used to control the rotational speed and/or position of the gantry 12 on the basis of imaging requirements. The control mechanism 30 may further include a transport table controller 36 which is configured to drive a transport table 28 to move to a suitable position, so as to position the subject to be examined in the gantry 12 to perform a scout scan or an axial scan or a bolt scan or other scan modes in order to acquire the projection data of the target volume of the subject to be examined. Furthermore, the carrier table 28 includes a driving apparatus, and the carrier table controller 36 may control the carrier table 28 by controlling the driving apparatus.

The system 10 further includes an image reconstruction module 50. As described above, the DAS 26 samples and digitizes the projection data acquired by the plurality of detector modules 20. Next, the image reconstruction module 50 performs high-speed image reconstruction on the basis of the aforementioned sampled and digitized projection data. In some embodiments, the image reconstruction module 50 stores the reconstructed image in a storage device or a mass memory 46. Or, the image reconstruction module 50 transmits the reconstructed image to a computer 40 to generate information for diagnosing and evaluating patients. For example, a first scout image and a diagnostic section image are generated on the basis of the projection data acquired by the scout scan or the axial scan or the bolt scan or the other scan modes.

Although the image reconstruction module 50 is illustrated as a separate entity in FIG. 16, in some embodiments, the image reconstruction module 50 may form a part of the computer 40. Or, the image reconstruction module 50 may not exist in the system 10, or the computer 40 may perform one or more functions of the image reconstruction module 50. Furthermore, the image reconstruction module 50 may be located at a local or remote location, and may be connected to the system 10 using a wired or wireless network. In some embodiments, computing resources having a centralized cloud network may be used for the image reconstruction module 50.

In some embodiments, the system 10 further includes the computer 40, wherein data sampled and digitized by the DAS 26 and/or an image reconstructed by the image reconstruction module 50 is transmitted to a computer or the computer 40 for processing. In some embodiments, the computer 40 stores the data and/or image in a storage device such as a mass memory 46. The mass memory 46 may include a hard disk drive, a floppy disk drive, a CD-read/write (CD-R/W) drive, a digital versatile disc (DVD) drive, a flash drive, and/or a solid-state storage apparatus. The processor in the computer 40 determines the predicted section image according to the first scout image.

In some embodiments, the computer 40 transmits the reconstructed image and/or other information to a display 42, the display 42 being communicatively connected to the computer 40 and/or the image reconstruction module 50. In some embodiments, the computer 40 may be connected to a local or remote display, printer, workstation and/or similar device, for example, connected to such devices of medical institutions or hospitals, or connected to a remote device by means of one or a plurality of configured wires or a wireless network such as the Internet and/or a virtual private network. For example, the display displays the predicted section image and the corresponding scanning parameters.

Furthermore, the computer 40 may provide commands and parameters to the DAS 26 and the control mechanism 30 (including the gantry controller 32, the X-ray controller 34, and the carrier table controller 36) on the basis of user provision and/or system definition, so as to control a system operation, such as data acquisition and/or processing. In some embodiments, the computer 40 controls system operation on the basis of user input. For example, the computer 40 may receive user input such as commands, scanning protocols and/or scanning parameters, by means of an operator console 48 connected thereto. The operator console 48 may include a keyboard (not shown) and/or touch screen to allow a user to input/select commands, scanning protocols and/or scanning parameters.

In some embodiments, the system 10 may include or be connected to a picture archiving and communication system (PACS) (not shown in the figure). In some embodiments, the PACS is further connected to a remote system such as a radiology information system, a hospital information system, and/or an internal or external network (not shown) to allow operators at different locations to provide commands and parameters and/or access image data.

The method or process described in the aforementioned embodiments may be stored as executable instructions in a non-volatile memory in a computing device of the system 10. For example, the computer 40 may include executable instructions in the non-volatile memory and may apply the medical image imaging method in the embodiments of the present application.

The computer 40 may be configured and/or arranged for use in different manners. For example, in some implementations, a single computer 40 may be used; and in other implementations, a plurality of computers 40 are configured to work together (for example, on the basis of distributed processing configuration) or separately, and each computer 40 is configured to process specific aspects and/or functions, and/or process data for generating models used only for a specific system 10. In some implementations, the computer 40 may be local (for example, in the same place as one or a plurality of systems 10, for example, in the same facility and/or the same local network); and in other implementations, the computer 40 may be remote, and thus only accessible by means of a remote connection (for example, by means of the Internet or other available remote access technologies). In a specific implementation, the computer 40 may be configured in a manner similar to that of cloud technology, and may be accessed and/or used in a manner substantially similar to that of accessing and using other cloud-based systems.

Once data is generated and/or configured, the data can be replicated and/or loaded into the medical system 10, which may be accomplished in different manners. For example, models may be loaded by means of a directional connection or link between the system 10 and the computer 40. In this regard, communication between different elements may be accomplished using an available wired and/or wireless connection and/or according to any suitable communication (and/or network) standard or protocol. Alternatively or additionally, the data may be indirectly loaded into the system 10. For example, the data may be stored in a suitable machine-readable medium (for example, a flash memory card), and then the medium is used to load the data into the system 10 (for example, by a user or an authorized personnel of the system on site); or the data may be downloaded to an electronic device (for example, a laptop) capable of local communication, and then the device is used on site (for example, by a user or an authorized personnel of the system) to upload the data to the system 10 by means of a direct connection (for example, a USB connector).

Further provided in the embodiments of the present application is a computer-readable program useful for understanding the invention. When executed, the program causes a computer to perform the medical imaging method described in the aforementioned embodiments in the apparatus, system or computer device.

Further provided in the embodiments of the present application is a storage medium storing a computer-readable program useful for understanding the invention. The computer-readable program causes a computer to perform the medical imaging method described in the aforementioned embodiments in the apparatus, system or computer device.

The above apparatus and method of the present application can be implemented by hardware, or can be implemented by hardware in combination with software. The present application relates to the foregoing type of computer-readable program. When executed by a logic component, the program causes the logic component to implement the foregoing apparatus or a constituent component, or causes the logic component to implement various methods or steps as described above. The present application further relates to a storage medium for storing the above program, such as a hard disk, a disk, an optical disk, a DVD, a flash memory, etc.

The method/apparatus described in view of the embodiments of the present application may be directly embodied as hardware, a software module executed by a processor, or a combination of the two. For example, one or more of the functional block diagrams and/or one or more combinations of the functional block diagrams shown in the drawings may correspond to either respective software modules or respective hardware modules of a computer program flow. The foregoing software modules may respectively correspond to the steps shown in the figures. The foregoing hardware modules can be implemented, for example, by firming the software modules using a field-programmable gate array (FPGA).

The software modules may be located in a RAM, a flash memory, a ROM, an EPROM, an EEPROM, a register, a hard disk, a portable storage disk, a CD-ROM, or any other form of storage medium known in the art. The storage medium may be coupled to a processor, so that the processor can read information from the storage medium and can write information into the storage medium. Alternatively, the storage medium may be a constituent component of the processor. The processor and the storage medium may be located in an ASIC. The software module may be stored in a memory of a mobile terminal, and may also be stored in a memory card that can be inserted into a mobile terminal. For example, if a device (such as a mobile terminal) uses a large-capacity MEGA-SIM card or a large-capacity flash memory device, the software modules can be stored in the MEGA-SIM card or the large-capacity flash memory apparatus.

One or more of the functional blocks and/or one or more combinations of the functional blocks shown in the accompanying drawings may be implemented as a general-purpose processor, a digital signal processor (DSP), an application-specific integrated circuit (ASIC), a field-programmable gate array (FPGA) or other programmable logic devices, discrete gate or transistor logic devices, a discrete hardware assembly, or any appropriate combination thereof for implementing the functions described in the present application. One or a plurality of the functional blocks and/or one or a plurality of combinations of the functional blocks described relative to the figures may also be implemented

as a combination of computing devices, such as a combination of a DSP and a microprocessor, a plurality of microprocessors, one or a plurality of microprocessors in communication combination with a DSP, or any other such configuration. The above embodiments merely provide illustrative descriptions of the embodiments of the present application. However, the present application is not limited thereto, and appropriate variations may be made on the basis of the above embodiments. For example, each of the above embodiments may be used independently, or one or more among the above embodiments may be combined.

The present application is described above with reference to specific embodiments. However, it should be clear to those skilled in the art that the foregoing description is merely illustrative and is not intended to limit the scope of protection of the present application. Various variations and modifications may be made by those skilled in the art according to the scope of the present application as defined by the appended claims, and these variations and modifications also fall within the scope of the present application.

Preferred embodiments of the present application are described above with reference to the accompanying drawings. Many features and advantages of the implementations are clear according to the detailed description, and therefore the appended claims are intended to cover all these features and advantages that fall within the scope of these implementations. In addition, as many modifications and changes could be easily conceived of by those skilled in the art, the embodiments of the present application are not limited to the illustrated and described precise structures and operations, but can encompass all appropriate modifications, changes, and equivalents that fall within the scope of the appended claims.

## Claims

1. A medical imaging method comprising:
determining (201) the position of a medical accessory (300) in a reference coordinate system based on a camera image (1100) acquired by an auxiliary camera (200);
reconstructing (202) a medical image (901) according to a scanning result and based on the reference coordinate system; and
displaying in real time the position of the medical accessory in the medical image;
**characterized in that** the method further comprises:
generating a grid matrix (1102) according to the scanning result, wherein a starting point and/or a target point of a planned movement path of the medical accessory are/is marked in the grid matrix; and
superimposing the grid matrix in the camera image.

2. The method according to claim 1, wherein
the reference coordinate system comprises a world coordinate system, and the origin of the world coordinate system is located at the center of a scanning gantry (101).

3. The method according to claim 1, further comprising:
displaying on the grid matrix in real time the position of the medical accessory and/or information indicating the relative positional relationship between the medical accessory and the starting point and/or the target point.

4. The method according to claim 1, wherein
the medical image comprises a three-dimensional image and/or a two-dimensional image displayed on one or a plurality of planes.

5. The method according to claim 1, wherein the medical image comprises a two-dimensional image displayed on one or a plurality of planes, the method further comprising:
displaying in real time, according to the position of the medical accessory, the two-dimensional image corresponding to the position of the medical accessory.

6. The method according to claim 5, wherein the two-dimensional image corresponding to the position of the medical accessory comprises:
a first two-dimensional image that passes through the position of the medical accessory, and/or a second two-dimensional image that passes through the target point of the medical accessory, and that is perpendicular to the first two-dimensional image passing through the position of the medical accessory, wherein:
the second two-dimensional image comprises marking information of the target point, wherein the marking information comprises information related to the relative positional relationship between the medical accessory and the target point.

7. The method according to claim 1, wherein the medical image comprises a three-dimensional image, the method further comprising:
in the three-dimensional image of the medical image, performing superimposed display of: the planned movement path of the medical accessory, and/or information (1103) indicating the relative positional relationship between the medical accessory and the starting point and/or the target point of the movement path of the medical accessory, and/or a predicted path determined according to the position and the extension direction of the medical accessory, and/or the camera image.

8. The method according to claim 1, wherein determining the position of a medical accessory in a reference coordinate system based on a camera image acquired by an auxiliary camera comprises:
determining (401) the position of the medical accessory in a camera coordinate system according to the camera image; and
determining (402) the position of the medical accessory in the reference coordinate system according to the position of the medical accessory in the camera coordinate system and a first transformation matrix between the camera coordinate system and the reference coordinate system.

9. The method according to claim 8, wherein
the medical accessory comprises a puncture needle and a marker; and
determining the position of the medical accessory in a camera coordinate system according to the camera image comprises:
recognizing (501) the marker in the camera image, and determining the extension direction and position information of the marker in the camera coordinate system; and
determining (502) the position of the puncture needle in the camera coordinate system according to the extension direction, the position information, and the relative positional relationship between the marker and the puncture needle.

10. The method according to claim 9, wherein
the extension direction of the marker forms a preset included angle with the extension direction of the puncture needle, and/or the marker is fixed at a preset position of the puncture needle; and/or the marker carries information related to the medical accessory.

11. The method according to claim 10, wherein
the marker is a cannula sleeved on the peripheral side of the puncture needle.

12. The method according to claim 9, wherein the marker comprises at least one of the following:
a component having a preset temperature, a component having a preset shape and/or pattern, or a component emitting light according to a preset rule.

13. The method according to claim 1, wherein reconstructing a medical image according to a scanning result and based on the reference coordinate system comprises:
reconstructing the medical image in the reference coordinate system according to the position of the scanning result in a scanning coordinate system and a second transformation matrix between the scanning coordinate system and the reference coordinate system.

14. The method according to claim 13, further comprising:
when a scanned subject moves from a first position in which scanning is performed to a second position, further reconstructing the medical image according to the relative positional relationship between the first position and the second position.

15. A storage medium storing a computer-readable program, said program configured to cause a computer to perform the method according to any of claims 1-14; or
a computer device configured to carry out the method according to any of claims 1-14.

## Patentansprüche

1. Medizinisches Bildgebungsverfahren, umfassend:
Bestimmen (201) der Position eines medizinischen Zubehörs (300) in einem Referenzkoordinatensystem basierend auf einem Kamerabild (1100), das durch eine Hilfskamera (200) erfasst wird;
Rekonstruieren (202) eines medizinischen Bildes (901) gemäß einem Abtastergebnis und basierend auf dem Referenzkoordinatensystem; und
Anzeigen der Position des medizinischen Zubehörs im medizinischen Bild in Echtzeit; **dadurch gekennzeichnet, dass** das Verfahren ferner umfasst:
Erzeugen einer Rastermatrix (1102) gemäß dem Abtastergebnis, wobei ein Startpunkt und/oder ein Zielpunkt einer geplanten Bewegungsbahn des medizinischen Zubehörs in der Rastermatrix markiert sind/ist; und
Überlagern der Rastermatrix im Kamerabild.

2. Verfahren nach Anspruch 1, wobei
das Referenzkoordinatensystem ein Weltkoordinatensystem umfasst und der Nullpunkt des Weltkoordinatensystems sich in der Mitte einer Abtastgantry (101) befindet.

3. Verfahren nach Anspruch 1, ferner umfassend:
Anzeigen der Position des medizinischen Zubehörs und/oder von Informationen, die die relative Positionsbeziehung zwischen dem medizinischen Zubehör und dem Startpunkt und/oder dem Zielpunkt angeben, in Echtzeit auf der Rastermatrix.

4. Verfahren nach Anspruch 1, wobei
das medizinische Bild ein dreidimensionales Bild und/oder ein zweidimensionales Bild umfasst, das auf einer oder mehreren Ebenen angezeigt wird.

5. Verfahren nach Anspruch 1, wobei das medizinische Bild ein zweidimensionales Bild umfasst, das auf einer oder mehreren Ebenen angezeigt wird, wobei das Verfahren ferner umfasst:
Anzeigen des zweidimensionalen Bildes, das der Position des medizinischen Zubehörs entspricht, in Echtzeit gemäß der Position des medizinischen Zubehörs.

6. Verfahren nach Anspruch 5, wobei das zweidimensionale Bild, das der Position des medizinischen Zubehörs entspricht, umfasst:
ein erstes zweidimensionales Bild, das durch die Position des medizinischen Zubehörs geht, und/oder ein zweites zweidimensionales Bild, das durch den Zielpunkt des medizinischen Zubehörs geht und das senkrecht zum ersten, durch die Position des medizinischen Zubehörs gehenden zweidimensionalen Bild verläuft, wobei:
das zweite zweidimensionale Bild Markierungsinformationen des Zielpunkts umfasst, wobei die Markierungsinformationen Informationen umfassen, die sich auf die relative Positionsbeziehung zwischen dem medizinischen Zubehör und dem Zielpunkt beziehen.

7. Verfahren nach Anspruch 1, wobei das medizinische Bild ein dreidimensionales Bild umfasst, wobei das Verfahren ferner umfasst:
im dreidimensionalen Bild des medizinischen Bildes Durchführen einer überlagerten Anzeige von: der geplanten Bewegungsbahn des medizinischen Zubehörs und/oder Informationen (1103), die die relative Positionsbeziehung zwischen dem medizinischen Zubehör und dem Startpunkt und/oder dem Zielpunkt der Bewegungsbahn des medizinischen Zubehörs angeben, und/oder einer vorhergesagten Bahn, die gemäß der Position und der Erstreckungsrichtung des medizinischen Zubehörs bestimmt wird, und/oder dem Kamerabild.

8. Verfahren nach Anspruch 1, wobei das Bestimmen der Position eines medizinischen Zubehörs in einem Referenzkoordinatensystem basierend auf einem Kamerabild, das durch eine Hilfskamera erfasst wird, umfasst:
Bestimmen (401) der Position des medizinischen Zubehörs in einem Kamerakoordinatensystem gemäß dem Kamerabild; und
Bestimmen (402) der Position des medizinischen Zubehörs im Referenzkoordinatensystem gemäß der Position des medizinischen Zubehörs im Kamerakoordinatensystem und einer ersten Transformationsmatrix zwischen dem Kamerakoordinatensystem und dem Referenzkoordinatensystem.

9. Verfahren nach Anspruch 8, wobei
das medizinische Zubehör eine Punktionsnadel und einen Marker umfasst; und
das Bestimmen der Position des medizinischen Zubehörs in einem Kamerakoordinatensystem gemäß dem Kamerabild umfasst:
Erkennen (501) des Markers im Kamerabild und Bestimmen der Erstreckungsrichtung und Positionsinformationen des Markers im Kamerakoordinatensystem; und
Bestimmen (502) der Position der Punktionsnadel im Kamerakoordinatensystem gemäß der Erstreckungsrichtung, den Positionsinformationen und der relativen Positionsbeziehung zwischen dem Marker und der Punktionsnadel.

10. Verfahren nach Anspruch 9, wobei
die Erstreckungsrichtung des Markers mit der Erstreckungsrichtung der Punktionsnadel einen vorgegebenen eingeschlossenen Winkel bildet und/oder der Marker an einer vorgegebenen Position der Punktionsnadel fixiert ist; und/oder der Marker Informationen über das medizinische Zubehör trägt.

11. Verfahren nach Anspruch 10, wobei
es sich bei dem Marker um eine auf der Umfangsseite der Punktionsnadel umhüllte Kanüle handelt.

12. Verfahren nach Anspruch 9, wobei der Marker mindestens eines der Folgenden umfasst:
eine Komponente mit einer vorgegebenen Temperatur, eine Komponente mit einer vorgegebenen Form und/oder einem vorgegebenen Muster oder eine Komponente, die Licht gemäß einer vorgegebenen Regel emittiert.

13. Verfahren nach Anspruch 1, wobei das Rekonstruieren eines medizinischen Bildes gemäß einem Abtastergebnis und basierend auf dem Referenzkoordinatensystem umfasst:
Rekonstruieren des medizinischen Bildes im Referenzkoordinatensystem gemäß der Position des Abtastergebnisses in einem Abtastkoordinatensystem und einer zweiten Transformationsmatrix zwischen dem Abtastkoordinatensystem und dem Referenzkoordinatensystem.

14. Verfahren nach Anspruch 13, ferner umfassend:
wenn sich ein abgetastetes Objekt aus einer ersten Position, in der das Abtasten durchgeführt wird, in eine zweite Position bewegt, weiteres Rekonstruieren des medizinischen Bildes gemäß der relativen Positionsbeziehung zwischen der ersten Position und der zweiten Position.

15. Speichermedium, das ein computerlesbares Programm speichert, wobei das Programm dazu ausgelegt ist, einen Computer zu veranlassen, das Verfahren nach einem der Ansprüche 1 bis 14 durchzuführen; oder
Computervorrichtung, die dazu ausgelegt ist, das Verfahren nach einem der Ansprüche 1 bis 14 auszuführen.

## Revendications

1. Procédé d'imagerie médicale comprenant :
la détermination (201) de la position d'un accessoire médical (300) dans un système de coordonnées de référence sur la base d'une image de caméra (1100) acquise par une caméra auxiliaire (200) ;
la reconstruction (202) d'une image médicale (901) selon un résultat de balayage et sur la base du système de coordonnées de référence ; et
l'affichage en temps réel de la position de l'accessoire médical sur l'image médicale ; **caractérisé en ce que** le procédé comprend en outre :
la génération d'une matrice quadrillée (1102) selon le résultat de balayage, dans laquelle un point de départ et/ou un point cible d'une trajectoire de mouvement planifiée de l'accessoire médical sont marqués dans la matrice quadrillée ; et
la superposition de la matrice quadrillée dans l'image de la caméra.

2. Procédé selon la revendication 1, dans lequel le système de coordonnées de référence comprend un système de coordonnées mondiales, et l'origine du système de coordonnées mondiales est située au centre d'un portique de balayage (101).

3. Procédé selon la revendication 1, comprenant en outre :
l'affichage en temps réel, sur la matrice quadrillée, de la position de l'accessoire médical et/ou d'informations indiquant la relation de position relative entre l'accessoire médical et le point de départ et/ou le point cible.

4. Procédé selon la revendication 1, dans lequel l'image médicale comprend une image tridimensionnelle et/ou une image bidimensionnelle affichée sur un plan ou une pluralité de plans.

5. Procédé selon la revendication 1, dans lequel l'image médicale comprend une image bidimensionnelle affichée sur un plan ou une pluralité de plans, le procédé comprenant en outre :
l'affichage en temps réel, selon la position de l'accessoire médical, de l'image bidimensionnelle correspondant à la position de l'accessoire médical.

6. Procédé selon la revendication 5, dans lequel l'image bidimensionnelle correspondant à la position de l'accessoire médical comprend :
une première image bidimensionnelle qui passe par la position de l'accessoire médical, et/ou une seconde image bidimensionnelle qui passe par le point cible de l'accessoire médical, et qui est perpendiculaire à la première image bidimensionnelle passant par la position de l'accessoire médical, dans lequel :
la seconde image bidimensionnelle comprend des informations de marquage du point cible, les informations de marquage comprenant des informations relatives à la relation de position relative entre l'accessoire médical et le point cible.

7. Procédé selon la revendication 1, dans lequel l'image médicale comprend une image tridimensionnelle, le procédé comprenant en outre :
dans l'image tridimensionnelle de l'image médicale, la réalisation de l'affichage superposé de : la trajectoire de mouvement planifiée de l'accessoire médical, et/ou des informations (1103) indiquant la relation de position relative entre l'accessoire médical et le point de départ et/ou le point cible de la trajectoire de mouvement de l'accessoire médical, et/ou une trajectoire prédite déterminée selon la position et de la direction d'extension de l'accessoire médical, et/ou l'image de la caméra.

8. Procédé selon la revendication 1, dans lequel la détermination de la position d'un accessoire médical dans un système de coordonnées de référence sur la base d'une image de caméra acquise par une caméra auxiliaire comprend :
la détermination (401) de la position de l'accessoire médical dans un système de coordonnées de caméra selon l'image de la caméra ; et
la détermination (402) de la position de l'accessoire médical dans le système de coordonnées de référence selon la position de l'accessoire médical dans le système de coordonnées de la caméra et une première matrice de transformation entre le système de coordonnées de la caméra et le système de coordonnées de référence.

9. Procédé selon la revendication 8, dans lequel
l'accessoire médical comprend une aiguille de ponction et un repère ; et
la détermination de la position de l'accessoire médical dans un système de coordonnées de caméra selon l'image de la caméra comprend :
la reconnaissance (501) du repère dans l'image de la caméra, et la détermination de la direction d'extension et d'informations de position du repère dans le système de coordonnées de la caméra ; et
la détermination (502) de la position de l'aiguille de ponction dans le système de coordonnées de la caméra selon la direction d'extension, les informations de position, et la relation de position relative entre le repère et l'aiguille de ponction.

10. Procédé selon la revendication 9, dans lequel la direction d'extension du repère forme un angle inclus prédéfini avec la direction d'extension de l'aiguille de ponction, et/ou le repère est fixé en une position prédéfinie de l'aiguille de ponction ; et/ou le repère porte des informations relatives à l'accessoire médical.

11. Procédé selon la revendication 10, dans lequel le repère est une canule emmanchée sur le côté périphérique de l'aiguille de ponction.

12. Procédé selon la revendication 9, dans lequel le repère comprend au moins l'un des éléments suivants :
un composant ayant une température prédéterminée, un composant ayant une forme et/ou un motif prédéfinis, et un composant émettant de la lumière selon une règle préétablie.

13. Procédé selon la revendication 1, dans lequel la reconstruction d'une image médicale selon un résultat de balayage et sur la base du système de coordonnées de référence comprend :
la reconstruction de l'image médicale dans le système de coordonnées de référence selon la position du résultat de balayage dans un système de coordonnées de balayage et une seconde matrice de transformation entre le système de coordonnées de balayage et le système de coordonnées de référence.

14. Procédé selon la revendication 13, comprenant en outre :
lorsqu'un sujet balayé passe d'une première position dans laquelle le balayage est effectué à une seconde position, la reconstruction supplémentaire de l'image médicale selon la relation de position relative entre la première position et la seconde position.

15. Support de stockage stockant un programme lisible par ordinateur, ledit programme étant configuré pour amener un ordinateur à réaliser le procédé selon l'une quelconque des revendications 1 à 14 ; ou
dispositif informatique configuré pour mettre en œuvre le procédé selon l'une quelconque des revendications 1 à 14.
